# EUROPEAN PATENT APPLICATION

(11) **EP 3 305 333 A1**
(43) Date of publication of application: **11.04.2018**
(21) Application number: 16192260.4
(22) Date of filing: 04.10.2016
(51) Int. Cl.: A61L 9/02, C11C 5/00

(54) **MELTABLE SCENTED COMPOSITION**

(71) Applicant: Eurvest S.A., 1400 Nivelles (BE)
(72) Inventor: Dmitruk, Aleksandra, 20-461 Lublin (PL); Luciani, Alain, 13390 AURIOL (FR)
(74) Representative: August Debouzy

(57) **Abstract**

The invention provides a meltable wax composition comprising at least one triglyceride of a fatty acid, at least a wax, at least an oil, at least a fragrance composition. The invention also provides a use and a process for diffusion of the said composition.

## Description

### FIELD OF THE INVENTION

The present invention relates to a meltable scented composition, and to its use for dispensing fragrance into the surrounding environment.

### TECHNICAL BACKGROUND

Candles have been used for centuries to provide illumination for the surrounding area. In more recent years, candles have been used as a fragrancing and/or deodorizing mechanism for the home. A typical candle includes a wax body with a wick extending therethrough. Alternatively, meltable scented compositions, also known as wax melts, can be placed in a warmer. Generally, users can warm waxes and oils to provide desired effects to the surrounding environment.

Traditional warmers and meltable scented compositions have some drawbacks. One main drawback is that when the fragrance is gone, the user needs to remove the melted yet hardened composition from the warmer which might be really problematic since the hardened composition is usually very much attached to the warmer.

### TECHNICAL BACKGROUND

US8809744 discloses a wax remover for removing solidified wax within a wax container of a scent-warmer device.

WO2016/014172 discloses a device and method of supporting a meltable wax tart and removing of hot molten wax.

FR2847477 discloses a method of fragrance delivery in ambient air and a device for its implementation, wherein the product is composed, in weight, of 30% of odorant substance, 50% petroleum jelly, 10% of white beeswax and 10% of stearin.

EP1632251 discloses a wax composition comprising stearates of alkaline and alkaline-earth metals or sodium stearate.

WO2008/141777 discloses a composition suitable for use as an air freshener or candle, containing a fragrance composition, wherein the organic phase is structured by a structuring polymer which is end capped with a fatty alcohol or fatty acid, notably isostearyl alcohol or isostearic acid.

### SUMMARY OF THE INVENTION

The invention provides a meltable scented composition comprising at least one triglyceride of a fatty acid, at least a wax, at least an oil, at least a fragrance composition.

According to an embodiment the tryglyceride is stearin.

According to a further embodiment the composition comprises from 20 to 60%, preferably from 30 to 50%, more preferably from 35 to 45% by weight, based on the total weight of the composition, of tryglyceride.

According to a further embodiment the wax is palm wax.

According to a further embodiment the composition comprises from 20 to 40%, preferably from 25 to 35%, by weight, based on the total weight of the composition, of wax.

According to a further embodiment the oil is paraffinic oil.

According to a further embodiment the composition comprises from 10 to 30%, preferably from 15 to 25%, by weight, based on the total weight of the composition, of oil.

According to a further embodiment the composition comprises from 5 to 15%, preferably from 8 to 12%, by weight, based on the total weight of the composition, of fragrance.

According to a further embodiment the composition comprises by weight, based on the total weight of the composition:
- from 20 to 60%, preferably from 30 to 50%, more preferably 35 to 45%, of stearin,
- from 20 to 40%, preferably from 25 to 35%, of palm wax,
- from 10 to 30%, preferably from 15 to 25%, of paraffinic oil, and
- from 5 to 15%, preferably from 8 to 12%, of fragrance.

According to a further embodiment the composition further comprises a hyperbranched polyolefin, preferably in an amount from 0.1 to 5%, preferably from 0.25 to 3% by weight, based on the total weight of the composition.

According to a further embodiment the composition has a melting temperature from 45°C to 65°C, preferably from 50°C to 60°C.

According to a further embodiment the composition is free of silicone.

The invention also provides for a process for the diffusion of a fragrance, comprising the step of placing the meltable scented composition, heating said composition until it melts and allowing the fragrance to diffuse. The invention also provides for the use of the composition of the invention to release a fragrance upon heating and melting thereof.

The invention further provides advantages as will be apparent from the description. The main advantage of the composition over the compositions of the prior art is that they are non adhesive, i.e. can be removed easily by the user from the warmer.

### DESCRIPTION OF EMBODIMENTS OF THE INVENTION

The invention is now disclosed in more details in the following description. Unless specified otherwise, amounts and percentages are by weight, based on the total weight of the composition.

### Triglyceride.

As known in the art, triglycerides are fatty acid esters of glycerol. The fatty acids may be plant source or other source.

While stearin is more preferred, it should be recognized that other TGs are suitable and included within the present invention.

Stearin is a triglyceride derived from three units of stearic acid. Stearic acid is a saturated fatty acid with an 18-carbon chain. Stearic acid is obtained from fats and oils by the saponification of the triglycerides using hot water (above 200 °C). The resulting mixture is then disti lied.

Stearin is well known and has been used as a hardening agent in the manufacture of candles and soap. It raises the bending (softening) point and lowers the melting point.

The overall amount of triglyceride in the composition is typically from 20 to 60%, preferably from 30 to 50%, more preferably from 35 to 45% by weight. Wax

As stated above, wax is present in the composition. Suitable waxes are natural waxes such as beeswax, lanolin, palm wax and synthetic waxes (hydrocarbon waxes) such as paraffin wax. Palm wax is preferred. A mixture of two or more waxes is also possible; it can be a mixture of at least one natural wax and at least one synthetic wax, or a mixture of at least two synthetic or a mixture of at least two natural waxes. A possible mixture is a mixture of palm wax and paraffin wax, according to varying ratios, e.g. from 5:1 to 1:5, preferably 1:2 to 3:1.

The overall amount of wax is typically from 20 to 40%, preferably from 25 to 35%, by weight, based on the total weight of the composition.

While palm wax is more preferred, it should be recognized that other vegetal oil are suitable and included within the present invention. Palm wax is well known, and has been used in candles for many years. Palm wax consists mostly of aliphatic esters (about 40 wt%), diesters of 4-hydroxycinnamic acid (about 21.0 wt%), ω-hydroxycarboxylic acids (13.0 wt%), and fatty acid alcohols (12 wt%). The compounds are predominantly derived from acids and alcohols in the C26-C30 range.

A specific wax, microcrystalline wax can be added to the composition. It is known as an improver and is available as product

### Oil.

As known in the art, oil is any neutral, nonpolar chemical substance that is a viscous liquid at ambient temperatures and is both hydrophobic and lipophilic. While mineral oil, and more particularly paraffin oil, is more preferred, it should be recognized that other vegetal oil are suitable and included within the present invention. Mineral oil is any of various colorless, odorless, light mixtures of higher alkanes from a mineral source, particularly a distillate of petroleum.

The overall amount of oil is typically from 10 to 30%, preferably from 15 to 25%, by weight, based on the total weight of the composition.

### Fragrance

As used herein the term "fragrance" is used to indicate any odoriferous material that is available in a generally liquid carrier. The fragrance will most often be liquid at about 25° C. A wide variety of c hemicals are known for fragrance uses, including materials such as aldehydes, ketones, and esters. More commonly, naturally occurring plant and animal oils are known for use as fragrances. Typical fragrances can comprise, for example, woody/earthy bases containing exotic materials such as sandalwood, civet and patchouli oil. The fragrances can be of a light floral fragrance, e.g. rose extract, violet extract, lilac and the like. The fragrances can also be formulated to provide desirable fruity odors, e.g. lime, lemon, and orange.

The overall amount of fragrance is typically from 5 to 15%, preferably from 8 to 12%, by weight, based on the total weight of the composition. The amount of fragrance also includes the carrier, which can be an oil.

### Dye

A dye is typically present in the composition as is known in the art. Any dye may generally be used, subject to compatibility, i.e. it does not substantially change the properties or structure of the mixture, as is known to the skilled man. Liquid dyes or dyes that dissolve at temperatures of e.g. 60-80°C (above the melting temperature of the composition of the invention) are appropriate.

### Additive

The composition of the invention may comprise additives, especially a polymer. The polymer is a hyperbranched polyolefin and is available under the trademark Vybar™. Different grades are available, such as grades Vybar™ 103, Vybar™ 260, Vybar™ 343 and Vybar™ 825.

### Method of making.

The compositions of the present invention may be prepared by a variety of methods. One known method comprises placing all ingredients in one pot, heating until all ingredients are melted, stirring until a homogeneous mass is obtained, pouring into individual molds and cooling until tarts are obtained.

### Warmer.

The warmer may be of a traditional design such as is presently found in the art.

### Method of using.

When used, the composition of the present invention may simply be heated. Heating may be carried out by any means such as by placing a meltable scented tart in a warmer such as a ceramic warmer for instance. The length of time that the composition must be heated will depend on a number of factors, including the quantity of composition to be heated, the nature of the warmer in which it is held and the amount of fragrance that is incorporated. Suitable heating instructions may be provided in association with the warmer adapted to contain the meltable scented composition. Once the composition has been warmed, it will start release the fragrance in the surrounded environment.

The melting point of the composition is about 55°C, which is a temperature typical in the art.

The composition of the present invention are such that residues are removable from the warmer by the user with ease as the composition has anti adhesive properties. When the meltable scented composition is set to cool down in the warmer, it solidifies and it is not sticking to the warmer. The user can easily take it off and this prevent contamination of the warmer.

Unless specified to the contrary, all percentages are by weight based on the total weight of the meltable scented composition.

### EXAMPLES

The following further examples illustrate the invention without limiting it.

### Example 1

The following composition with % by weight is manufactured.

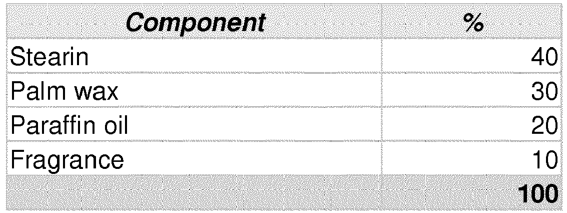

For preparation, an oil bath is heated and the components are heated and melted until complete homogenization by mixing using a lump breaker after melting.

The compositions is placed in a warmer and allowed to melt and diffuse the fragrance. Once solidified upon cooling, the hardened composition can be easily detached from the warmer, leaving substantially no residue on the surface of the warmer.

## Claims

1. A meltable scented composition comprising at least one triglyceride of a fatty acid, at least a wax, at least an oil, at least a fragrance composition.

2. The composition according to claim 1 wherein the tryglyceride is stearin.

3. The composition according to any one of the preceding claims comprising from 20 to 60%, preferably from 30 to 50%, more preferably from 35 to 45% by weight, based on the total weight of the composition, of tryglyceride.

4. The composition according to any one of the preceding claims wherein the wax is palm wax.

5. The composition according to any one of the preceding claims comprising from 20 to 40%, preferably from 25 to 35%, by weight, based on the total weight of the composition, of wax.

6. The composition according to any one of the preceding claims wherein the oil is paraffinic oil.

7. The composition according to any one of the preceding claims comprising from 10 to 30%, preferably from 15 to 25%, by weight, based on the total weight of the composition, of oil.

8. The composition according to any one of the preceding claims comprising from 5 to 15%, preferably from 8 to 12%, by weight, based on the total weight of the composition, of fragrance.

9. The composition according to any one of the preceding claims comprising by weight, based on the total weight of the composition:
- from 20 to 60%, preferably from 30 to 50%, more preferably 35 to 45%, of stearin,
- from 20 to 40%, preferably from 25 to 35%, of palm wax,
- from 10 to 30%, preferably from 15 to 25%, of paraffinic oil, and
- from 5 to 15%, preferably from 8 to 12%, of fragrance.

10. The composition according to any one of the preceding claims further comprising a dye, preferably in an amount up to 0.5% by weight, based on the total weight of the composition, of fragrance.

11. The composition according to any one of the preceding claims further comprising a hyperbranched polyolefin, preferably in an amount from 0.1 to 5%, preferably from 0.25 to 3% by weight, based on the total weight of the composition.

12. The composition according to any one of the preceding claims which has a melting temperature from 45°C to 65°C, prefer ably from 50°C to 60°C.

13. The composition according to any one of the preceding claims free of silicone.

14. Use of a composition according to any one of the preceding claims to release a fragrance upon heating and melting thereof.

15. A process for the diffusion of a fragrance, comprising the step of placing the meltable scented composition of any one of the preceding claims, heating said composition until it melts and allowing the fragrance to diffuse.
